# DEMANDE DE BREVET EUROPEEN

(11) **EP 3 753 713 A1**
(43) Date de publication de la demande: **23.12.2020**
(21) Numéro de dépôt: 20180376.4
(22) Date de dépôt: 16.06.2020
(51) Int. Cl.: B29C 65/20, B29C 65/78, A61M 39/14

(54) **ELÉMENT DE SUPPORT AVEC PORTION D'ISOLATION THERMIQUE**

(30) Priorité: 19.06.2019 FR 1906574
(71) Demandeur: MGA Technologies, 69380 Civrieux d'Azergues (FR)
(72) Inventeur: de MALLIARD, Hervé, 69160 Tassin la Demi-Lune (FR)
(74) Mandataire: Delorme, Nicolas

(57) **Abrégé**

Elément de support (1) pour une opération de thermo-soudure comportant un corps central présentant au moins un emplacement de réception (3) adapté pour recevoir un tube (4), ledit élément de support (1) étant caractérisé en ce qu'il comporte une portion isolante, fixée au corps central (2) et réalisée dans un matériau présentant une effusivité thermique inférieure à celle du matériau constituant le corps central.

## Description

La présente invention se rapporte à un élément de support d'au moins un tube déformable.

Elle concerne également une machine de thermo-soudure comportant un tel élément de support.

Dans les domaines pharmaceutique ou biomédical, il est connu de réaliser des opérations de thermo-soudure de tubes flexibles, par exemple des tubes en matière thermoplastique élastomère, dans lesquels peuvent circuler des flux de liquides.

Par exemple, une opération de connexion par thermo-soudure peut permettre de connecter entre eux deux contenants en réalisant une connexion bout-à-bout entre un premier tube relié au premier contenant et un deuxième tube relié au deuxième contenant.

De telles opérations de thermo-soudure sont habituellement réalisées à l'aide de machines de thermo-soudure comportant un dispositif chauffant capable de sectionner et/ou de faire fondre des tubes préalablement disposés sur des éléments de support.

Par exemple, le document EP2419257 décrit une machine permettant de connecter deux tubes flexibles positionnés sur deux éléments de supports grâce à une lame chauffante.

Cependant, la réalisation d'une telle opération de connexion nécessite de porter la lame chauffante à une température très élevée afin de sectionner et faire fondre les tubes à connecter, ces étapes de section et de fusion donnant lieu à une importante déperdition de chaleur de la lame chauffante vers son milieu environnant.

En particulier, lorsque la lame chauffante est placée à proximité des éléments de support maintenant les tubes en position, une part considérable de la chaleur de la lame chauffante est absorbée par ces derniers.

Il est ainsi nécessaire de compenser ces pertes énergétiques en chauffant davantage la lame chauffante, rendant l'opération de connexion moins efficace et plus coûteuse.

La présente invention a pour but de résoudre en tout ou partie cet inconvénient, en proposant un élément de support permettant le limiter les pertes thermiques lors d'une opération de thermo-soudure.

Un autre but de l'invention est de proposer un élément de support qui soit simple de conception et de fabrication.

Encore un autre but de l'invention est de proposer une machine de thermo-soudure utilisant un tel élément de support, présentant un meilleur rendement énergétique.

A cet effet, elle propose un élément de support pour une opération de thermo-soudure comportant un corps central présentant au moins un emplacement de réception adapté pour recevoir un tube, ledit élément de support étant caractérisé en ce qu'il comporte une portion isolante, fixée au corps central et réalisée dans un matériau présentant une effusivité thermique inférieure à celle du matériau constituant le corps central.

Un tel élément de support permet ainsi de maintenir en position, dans l'emplacement de réception, au moins un tube destiné à subir une opération de thermo-soudure, tout en permettant de limiter les échanges thermiques ayant lieu entre un dispositif chauffant intervenant au cours de ladite opération de thermo-soudure et lui-même.

En effet, la portion isolante a pour caractéristique de présenter une faible effusivité thermique, grandeur permettant de quantifier la rapidité avec laquelle la portion isolante absorbe de l'énergie thermique lorsque celle-ci est placée à proximité d'un objet plus chaud qu'elle.

Du fait de sa faible effusivité thermique, la portion isolante aura ainsi tendance à absorber très peu de calories en provenance du dispositif chauffant pendant toute la durée pendant laquelle elle est amenée à se trouver à proximité de ce dernier.

La présence de cette portion isolante a donc pour effet d'isoler thermiquement l'élément de support selon l'invention et de limiter la quantité d'énergie absorbée par cet élément de support pendant l'opération de thermo-soudure.

Cette portion isolante permet ainsi de rendre l'opération de thermo-soudure énergétiquement plus efficace et donc moins coûteuse à mettre en œuvre.

Il est à noter que, le corps central de l'élément de support pouvant être soumis à d'importants efforts mécaniques, le matériau dans lequel celui-ci est réalisé présente une effusivité thermique supérieure à celle de la portion isolante mais est avantageusement choisi pour ses caractéristiques de robustesse et solidité.

En effet, il est essentiel que l'élément de support ne puisse pas être aisément déformé, afin de pouvoir garantir un placement précis du ou des tubes dans l'emplacement de réception et la bonne qualité de l'opération de thermo-soudure.

Dans un mode de réalisation, le corps central présente deux faces longitudinales opposées, chacune de ces faces longitudinales opposées comportant un emplacement de réception adapté pour recevoir un tube.

L'élément de support est ainsi adapté pour recevoir et maintenir en position deux tubes parallèlement l'un à l'autre, par exemple en vue d'une opération de connexion de ces deux tubes bout à bout.

Selon une caractéristique, chaque emplacement de réception est formé par des éléments de guidage disposés en porte-à-faux sur l'une des faces longitudinales du corps central.

Selon une possibilité, le corps central présente deux faces transversales opposées joignant les faces longitudinales, la portion isolante étant fixée sur l'une de ces faces transversales opposées.

Cette disposition particulière de la portion isolante sur l'une des faces transversales du corps central est particulièrement adaptée à la réalisation d'une opération de section du ou des tubes positionnés dans les emplacements de réception.

En effet, il est ainsi possible de sectionner ces derniers par le déplacement d'une lame chauffante le long de cette face transversale : la lame chauffante fait alors face à la portion isolante disposée sur cette même face transversale pendant l'opération de section.

De la sorte, les pertes de chaleur émanant de la lame chauffante sont dirigées vers la face transversale de l'élément de support comportant la portion isolante : les flux de chaleur atteignant les zones de cette face transversale recouvertes par la portion isolante seront donc peu ou pas absorbés par l'élément de support.

Avantageusement, la portion isolante recouvre entièrement l'une des faces transversales opposées du corps central.

Cette caractéristique permet de limiter le plus possible la quantité de chaleur absorbée par l'élément de support, car cette quantité est proportionnelle à la proportion de la face transversale faisant face au dispositif chauffant recouverte par la portion isolante : plus cette proportion est importante, moins l'élément de support absorbe de chaleur.

Selon une possibilité, le matériau dans lequel la portion isolante est réalisée est un polymère thermoplastique de type PTFE (polytétrafluoroéthylène).

Par exemple, la portion isolante pourra être réalisée en Téflon, matériau présentant de bonnes propriétés de résistance thermique, et en particulier une très faible valeur d'effusivité thermique.

Le corps central pourra, lui, être réalisé dans un alliage métallique présentant une résistance thermique faible mais une bonne résistance aux efforts ou chocs mécaniques.

L'invention concerne également une machine de thermo-soudure comportant :
- un dispositif chauffant adapté pour faire fondre au moins partiellement au moins un tube, et
- au moins un élément de support tel que précédemment décrit, adapté pour recevoir ledit tube.

Dans un mode de réalisation, la machine de thermo-soudure comporte deux éléments de support tels que précédemment décrits, lesdits éléments de support étant disposés de manière que le dispositif chauffant puisse être positionné entre les portions isolantes respectives desdits éléments de support.

Selon une possibilité, les éléments de support sont adaptés pour recevoir deux tubes, et positionnés de sorte que leurs portions isolantes respectives soient en vis-à-vis, et le dispositif chauffant comporte une lame chauffante, adaptée pour sectionner lesdits tubes, déplaçable entre les portions isolantes respectives desdits éléments de support, ladite machine de thermo-soudure étant alors adaptée pour réaliser une opération de connexion desdits tubes par thermo-soudure.

Une telle machine de thermo-soudure présentera ainsi des pertes énergétiques faibles, car le dispositif chauffant est amené à se trouver positionné, au cours d'une opération de thermo-soudure, face à chacune des portions isolantes des éléments de support : la quantité de chaleur absorbée par les éléments de support correspond donc à la chaleur absorbée par chacune des portions isolantes de ces derniers.

De la sorte, du fait de la forte résistance thermique de ces mêmes portions isolantes, les échanges thermiques entre les éléments de support et le dispositif chauffant seront donc minimaux et la machine de thermo-soudure présente ainsi un meilleur rendement énergétique.

D'autres caractéristiques et avantages de la présente invention apparaîtront à la lecture de la description détaillée ci-après, d'un exemple de mise en œuvre non limitatif, faite en référence aux Figures annexées dans lesquelles :
[Fig. 1] est une vue en perspective d'un élément de support selon l'invention,
[Fig. 2] est une vue latérale d'un élément de support avec deux tubes en position,
[Fig. 3] est une vue de trois modes de réalisation d'un élément de support selon l'invention,
[Fig. 4] est une vue en perspective de deux tubes en position sur deux éléments de support en vue d'une opération de connexion, et
[Fig. 5] est une vue en perspective de deux éléments de support selon l'invention utilisés dans une machine de connexion.

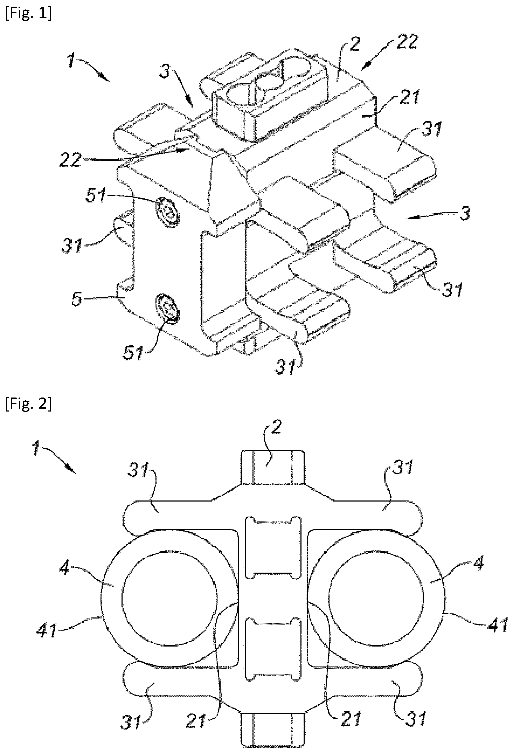

La figure 1 est une vue en perspective d'un élément de support 1 selon l'invention. Cet élément de support 1 comporte un corps central 2, présentant deux faces longitudinales 21 opposées et deux faces transversales 22 opposées.

L'élément de support 1 comporte sur chacune des faces longitudinales 21 un emplacement de réception 3, adapté pour recevoir un tube 4, comme illustré sur la figure 2.

Chaque emplacement de réception 3 est formé par des éléments de guidage 31 placés en porte-à-faux sur chacune des faces longitudinales 21 de l'élément de support 1.

Il est à noter que les dimensions des emplacements de réception 3 sont adaptées aux dimensions des tubes 4 qu'ils sont amenés à recevoir, de sorte que, lorsque qu'un tube 4 est positionné dans un emplacement de réception 3, la paroi externe 41 du tube 4 soit en contact avec les éléments de guidage 31 et l'une des faces longitudinales 21 du corps central 2.

Ainsi, chaque tube 4 est maintenu en position dans l'emplacement de réception 3 de l'élément de support 1 de manière fiable, en prévision d'une opération de thermo-soudure.

Il est par ailleurs à noter que les tubes 4 peuvent dépasser latéralement des emplacements de réception 3 dans lesquels ils sont positionnés, afin d'éventuellement permettre une déformation latérale de ceux-ci menant à leur obturation, en exerçant des efforts de compression mécanique sur leur paroi externe 41.

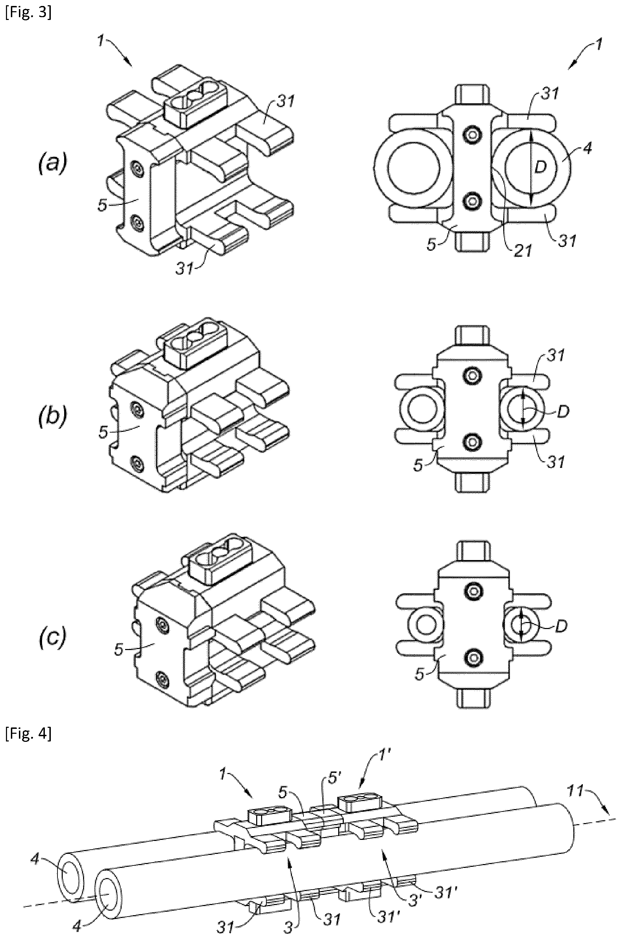

La figure 3 représente trois modes de réalisation (figures 3a, 3b, 3c) d'un élément de support 1, chacun de ces modes de réalisation étant adapté à un modèle particulier de tube à recevoir dans les emplacements de réception 3.

En effet, comme décrit plus haut, les dimensions des emplacements de réceptions doivent être choisies en fonction de celles des tubes qu'ils sont amenés à recevoir, afin de garantir leur bon positionnement et la qualité de l'opération de thermo-soudure qu'ils subiront.

En particulier, sur la figure 3, la distance D séparant les éléments de guidage 31 en vis-à-vis sur une même face longitudinale 21 est choisie pour correspondre au diamètre externe des tubes 4.

Ainsi, à chaque modèle de tube 4 existant est associé un modèle d'élément de support 1, dont la distance D séparant les éléments de guidage 31 correspond au diamètre externe du modèle particulier de tube 4.

Par ailleurs, l'élément de support 1 comporte également une portion isolante 5, fixée sur l'une des faces transversales 22 à l'aide de vis 51.

D'autres moyens de fixation de cette portion isolante 5 sont évidemment envisageables.

La portion 5 recouvre en totalité la face transversale 22 sur laquelle elle est fixée. Cette portion 5 a pour caractéristique de présenter une forte résistance thermique, et en particulier une effusivité thermique plus faible que le matériau dans lequel est réalisé le corps central 2 (et les éléments de guidage 31).

Cette propriété, quantifiant la faible propension de cette portion isolante 5 à absorber de la chaleur lorsque celle-ci est placée à proximité d'un objet plus chaud qu'elle, confère à la portion isolante 5 un caractère d'isolant thermique : si un objet chaud venait à être placé à proximité de la portion isolante 5, les échanges thermiques ayant lieu entre l'élément de support 1 et un tel objet chaud seront plus faibles que si l'élément de support 1 était dépourvu d'une telle portion isolante 5.

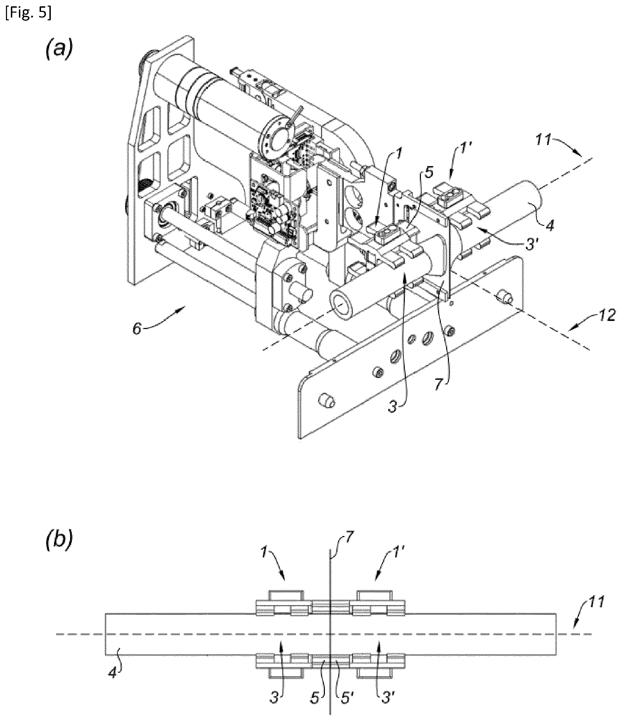

La présence de la portion isolante 5 permet ainsi limiter la quantité de chaleur absorbée par l'élément de support 1 au cours d'une opération de thermo-soudure, comme cela est illustré par les figures 4 et 5 suivantes.

La figure 4 représente deux tubes 4 placés dans les emplacements de réception respectifs 3 et 3' de deux éléments de support 1 et 1' selon l'invention.

Ces éléments de support 1 et 1' sont disposés l'un à côté de l'autre, de sorte que leurs portions isolantes respectives 5 et 5' se trouvent en vis-à-vis, séparées par une distance d'écartement E (non visible), et que les tubes 4 soient parallèles l'un à l'autre selon une direction longitudinale 11.

Cette configuration est particulièrement adaptée à la réalisation d'une opération de connexion grâce à une machine de connexion 6, visible sur la figure 5 et permettant de connecter bout à bout deux tubes 4.

Cette machine de connexion 6 comporte une lame chauffante 7 déplaçable selon une direction transversale 12 orthogonale à la direction longitudinale 11, entre une position escamotée et une position déployée (visible sur la figure 5a), et présentant une température élevée supérieure à la température de fusion des tubes 4 : dans sa position déployée, la lame chauffante 7 se trouve positionnée entre les deux éléments de support 1 et 1', sectionnant les tubes 4 dans la direction transversale 12 et entraînant leur fusion partielle.

Plus particulièrement, la lame chauffante sectionne les tubes 4 entre les portions isolantes 5 et 5'.

Il est à noter que, afin que cette configuration soit réalisable, il est nécessaire de choisir la distance d'écartement E de manière qu'elle soit supérieure à l'épaisseur de lame chauffante 7.

Du fait du caractère d'isolant thermique de ces portions isolantes 5 et 5', les échanges thermiques entre ces dernières et la lame chauffante sont très faibles : la quantité de chaleur, provenant de la lame chauffante 7, absorbée par les éléments de support 1 et 1' reste donc réduite.

Ainsi, la lame chauffante 7 dissipe moins d'énergie thermique vers son milieu environnant pendant l'opération de connexion, et il est possible de maintenir cette dernière à une température élevée en consommant moins d'énergie (par exemple, en consommant moins d'énergie électrique si la température de la lame chauffante 7 est augmentée par effet Joule, etc.).

L'utilisation des éléments de support 1 et 1' selon l'invention permet donc d'améliorer le rendement énergétique de la machine de connexion 6 et de rendre moins coûteuses les opérations de thermo-soudure effectuées grâce à celle-ci.

## Revendications

1. Elément de support (1,1') pour une opération de thermo-soudure comportant un corps central (2) présentant au moins un emplacement de réception (3,3') adapté pour recevoir un tube (4), ledit élément de support (1,1') étant **caractérisé en ce qu'**il comporte une portion isolante (5), fixée au corps central (2) et réalisée dans un matériau présentant une effusivité thermique inférieure à celle du matériau constituant le corps central (2).

2. Elément de support (1,1') selon la revendication précédente, dans lequel le corps central (2) présente deux faces longitudinales (21) opposées, chacune de ces faces longitudinales (21) opposées comportant un emplacement de réception (3, 3') adapté pour recevoir un tube (4).

3. Elément de support (1,1') selon la revendication précédente, dans lequel chaque emplacement de réception (3,3') est formé par des éléments de guidage (31,31') disposés en porte-à-faux sur l'une des faces longitudinales (21) du corps central (2).

4. Elément de support (1,1') selon l'une quelconque des revendications 2 et 3, dans lequel le corps central (2) présente deux faces transversales (22) opposées joignant les faces longitudinales (21), la portion isolante (5,5') étant fixée sur l'une de ces faces transversales (22) opposées.

5. Elément de support (1,1') selon la revendication précédente, dans lequel la portion isolante (5,5') recouvre entièrement l'une des faces transversales (22) opposées du corps central (2).

6. Elément de support (1,1') selon l'une quelconque des revendications précédentes, dans lequel le matériau dans lequel la portion isolante (5,5') est réalisée est un polymère thermoplastique de type PTFE (polytétrafluoroéthylène).

7. Machine de thermo-soudure (6) comportant :
- un dispositif chauffant adapté pour faire fondre au moins partiellement au moins un tube (4), et
- au moins un élément de support (1,1') conforme à l'une quelconque des revendications précédentes, adapté pour recevoir ledit tube (4).

8. Machine de thermo-soudure (6) selon la revendication précédente, comportant deux éléments de support (1,1') conformes à l'une quelconque des revendications 1 à 6 précédentes, lesdits éléments de support (1,1') étant disposés de manière que le dispositif chauffant puisse être positionné entre les portions isolantes (5,5') respectives desdits éléments de support (1,1').

9. Machine de thermo-soudure (6) selon la revendication précédente, dans laquelle :
- les éléments de support (1,1') sont conformes à la revendication 4 précédente, adaptés pour recevoir deux tubes (4), et positionnés de sorte que leurs portions isolantes (5,5') respectives soient en vis-à-vis, et
- le dispositif chauffant comporte une lame chauffante (7), adaptée pour sectionner lesdits tubes (4), déplaçable entre les portions isolantes (5,5') respectives desdits éléments de support (1,1'),
ladite machine de thermo-soudure (6) étant alors adaptée pour réaliser une opération de connexion desdits tubes (4) par thermo-soudure.
